# EUROPÄISCHE PATENTSCHRIFT

(11) **EP 1 893 554 B1**
(45) Veröffentlichungstag und Bekanntmachung des Hinweises auf die Patenterteilung: **04.06.2014**
(21) Anmeldenummer: 06743043.9
(22) Anmeldetag: 24.05.2006
(51) Int. Cl.: C07C 7/08

(54) **VERFAHREN ZUR GEWINNUNG VON REINSTYROL AUS EINER PYROLYSEBENZINFRAKTION**
METHOD FOR EXTRACTING PURE STYRENE FROM A PYROLYSIS BENZINE FRACTION
PROCEDE D'EXTRACTION DE STYRENE PUR D'UNE FRACTION DE PYROLYSE-BENZINE

(30) Priorität: 23.06.2005 DE 102005029643
(43) Veröffentlichungstag der Anmeldung: 05.03.2008
(73) Patentinhaber: ThyssenKrupp Industrial Solutions AG, 45143 Essen (DE)
(72) Erfinder: GEHRKE, Helmut, 59192 Bergkamen (DE)
(86) Internationale Anmeldenummer: PCT/EP2006/004918
(87) Internationale Veröffentlichungsnummer: WO 2006/136255

(56) Entgegenhaltungen:
- WO-A-2004/052492
- US-A- 5 849 982
- J C GENTRY AND C S KUMAR: "Generate more revenues from pygas processing" HYDROCARBON PROCESSING., Nr. June, 1997, Seiten 73-76, XP008072554 USGULF PUBLISHING CO. HOUSTON. in der Anmeldung erwähnt

## Beschreibung

Die Erfindung betrifft ein verbessertes Verfahren zur Gewinnung von Reinstyrol aus einer Pyrolysebenzinfraktion. Verfahren, die Reinstyrol mit Polymerisationsqualität aus Pyrolysebenzolfraktionen durch extraktive Destillation gewinnen, sind aus der US 3,684,665 von Toray und aus den Veröffentlichungen Sato, Hydrocarbon Processing, Mai 1973 Seite 141 ff, Morimoto et al, Bulletin of the Japan Petroleum Institute, Vol 16, No 1, Mai 1974 Seite 38 ff, Gentry et al, Hydrocarbon Processing, Juni 1997, Seite 73 ff., Emmrich et al., Int. J. of Hydrocarbon Engineering, Vol 3 No. 9 Oct. 1998, Seite 62 ff , und Gentry et al., Hydrocarbon Processing, Juni 2004, Seite 62 ff., bekannt.

Der übliche Verfahrensweg gemäß dem herkömmlichen Stand der Technik besteht aus den folgenden Schritten:
(1) Abtrennung einer C₇₋-Fraktion als Kopffraktion in einer ersten Destillationskolonne und Gewinnung einer C₈₊-Fraktion als Sumpffraktion,
(2) Abtrennung einer C₉₊-Fraktion im Sumpf einer zweiten Destillationskolonne und Gewinnung einer C₈-Fraktion am Kopf dieser Kolonne,
(3) Selektive Hydrierung von Phenylacetylen in der C₈-Fraktion,
(4) Zufuhr der selektiv-hydrierten C₈-Fraktion zu einer extraktiven Destillationskolonne und Ausführung der Destillation zur Gewinnung einer Styrol-Lösemittel-Fraktion im Sumpf und einer Styrol-armen Fraktion am Kopf der Kolonne,
(5) Nachbehandlung der Styrolfraktion zur Entfernung farbgebender Komponenten,
(6) Abschließende Aufbereitung der nachbehandelten Styrolfraktion zur Entfernung von Nebenprodukten, die bei der Nachbehandlung gebildet wurden.

Der Schritt (3) der selektiven Hydrierung von Phenylacetylen ist erforderlich, um diese Komponente aus dem Einsatzstrom zur extraktiven Destillation zu entfernen. Phenylacetylen (=Phenylethin) ist, ebenso wie Styrol eine ungesättigte Verbindung. Sein Siedepunkt liegt bei 142,4 °C und damit nahe dem Siedepunkt von Styrol mit 145,8 °C. Eine einfache destillative Abtrennung vom Styrol ist somit nicht möglich. Aufgrund seiner Polarität kann Phenylacetylen auch in einer extraktiven Destillation in Gegenwart eines selektiv wirkenden Lösemittels nicht vom Styrol getrennt werden, da die Affinität des Phenylacetylens zum selektiv wirkenden Lösemittel stärker ist als die Affinität des Styrols zu diesem Lösemittel. Dadurch würde das Phenylacetylen zusammen mit dem Styrol als Extrakt einer extraktiven Destillation anfallen.

J.C.Gentry und C.S. Kumar in Hydrocarbon Processing, Juni 1997, S.73-76, US Gulf Publishing Company Houston, beschreiben die Möglichkeiten der Rückgewinnung von Petrochemikalien aus Kraftstoffströmen und Pyrolysebenzinen durch extraktive Destillation. Erläutert werden insbesondere die Möglichkeiten der Rückgewinnung von Benzen, Toluen, Xylen, und Styren. Als geeignetes Mittel wird insbesondere die extraktive Destillation genannt, da viele der zu gewinnenden Komponenten nahe beieinander liegende Siedepunkte besitzen und durch einfache Destillation nicht zu trennen sind. Als besonders erstrebenswert wird die Verbesserung der Gewinnung von Styren beschreiben, da die für dieses Produkt bereitstehenden Gewinnungsverfahren bislang nur ungenügend profitabel sind. Als zu lösendes Problem wird das Vorhandensein von gleich oder nahezu gleichsiedenden Komponenten im Pyrolysebenzin beschrieben, wobei Beispiele genannt werden. Als einziger Stoff, der nicht durch die genannte extraktive Destillation entfernbar ist, wird Phenylacetylen genannt. Diese Verbindung kann durch selektive Hydrierung aus dem Stoffgemisch entfernt werden. Das erhaltene Styren besitzt eine Reinheit von 99,9 Gewichtsprozent.

Der Prozess einer extraktiven Destillation mit dem Ziel der Gewinnung von Styren aus Pyrolysebenzinen wird beispielhaft in der US 5849982 A beschrieben. Diese Schrift beschreibt ein Verfahren zur extraktiven Destillation zur Abtrennung von wenigstens einer substituierten ungesättigten aromatischen Verbindung aus einem Pyrolysebenzin, wobei dieses Pyrolysebenzin aromatische Verbindungen und mindestens ein aromatisches Verbindungspaar im Gemisch enthält, welches nahe beieinander liegende Siedepunkte besitzt, und ein extrahierendes Lösungsmittel aus zwei Bestandteilen gewählt wird, und der erste Bestandteil aus der Gruppe der Verbindungen Propylencarbonat, Sulfolan, Methylcarbinol, 1-Methyl-2-pyrrolidon, 2-Pyrollidon oder Gemischen daraus ausgewählt wird, und der zweite Bestandteil Wasser ist.

Die Spezifikationen für Rein-Styrol sehen vor, dass der Gehalt an Phenylacetylen im Reinstyrol einen bestimmten Grenzwert nicht überschreitet. Daher ist es notwendig, das Phenylacetylen aus dem Styrol-haltigen Strom vor der extraktiven Destillation zu entfernen. Eine Maßnahme der Entfernung ist die selektive Hydrierung (3). Bei diesem Verfahrensschritt wird das Phenylacetylen durch Wasserstoff in Styrol und/oder Ethylbenzol umgewandelt. Eine typische Ausführung dieser Verfahrensstufe hydriert das Phenylacetylen in der Flüssigphase unter erhöhtem Druck bei milden Temperaturbedingungen. Weiterhin wird für diesen Schritt ein Katalysator benötigt. Üblicherweise bedient sich der Fachmann dabei eines Edelmetall-Katalysators (z. B. Pt oder Pd), welcher auf einem Träger (z. B. Al₂O₃, SiO₂ oder TiO₂) aufgebracht wurde.

Die Hydrierbedingungen hinsichtlich Druck, Temperatur, Verweilzeit und Katalysatormenge werden dahingehend optimiert, dass der Gehalt an Phenylacetylen im Effluent des Reaktors so gering ist, so dass bei der weiteren Styrol-Gewinnung der verbleibende Phenylacetylengehalt im Styrol den geforderten Grenzwert nicht überschritten wird und der Styrolverlust aufgrund von Überhydrierung minimiert wird.

Für die Nachbehandlung nach Schritt (5) sind aus der US 3,763,015 die Behandlung mit 60%-iger Salpetersäure HNO₃ und aus der DE 198 53 916 die Behandlung mit Maleinsäureanhydrid (MSA) bekannt. Nachteilig bei diesen beiden Verfahren ist jedoch, dass überhaupt weitere Chemikalien zur Nachbehandlung erforderlich sind, ferner der hohe apparative Aufwand für die erforderlichen Reaktionsbehälter und Aufarbeitungsschritte und der durch die Behandlung mit HNO₃ bzw. MSA erhöhte Verlust an Styrol, der überwiegend durch Styrolpolymerisation bedingt ist.

Die Nachbehandlung ist erforderlich, weil das mit dem Schritt (4) gewonnene Styrolprodukt zwar in Bezug auf die noch enthaltenen Verunreinigungen alle aus der ASTM D-2827 vorgegebenen Anforderungen erfüllt, nicht jedoch die Anforderung an die Farbe des Produktes. Dies lehren insbesondere die Veröffentlichungen von Sato 1973, Morimoto 1974 und von Emmrich et al. 1998. Obige ASTM-Norm fordert eine Farbe von kleiner oder gleich 10 mg Pt/l (nach ASTM D-1209). Das aus der extraktiven Destillation erhaltene Styrol-Produkt ist jedoch, je nach Herkunft des Pyrolysebenzins, leicht bis stark gelb gefärbt. Diese Färbung beruht im wesentlichen auf der Anwesenheit von 1,3- Cyclopentadien-Derivaten oder anderen mehrfach ungesättigten, konjugierten Systemen.

Beispiele für diese Stoffgruppe sind:
5-Methylen-1,3-cyclopentadien (Fulven) 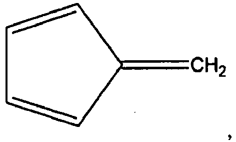
5-Ethyliden-1,3-cyclopentadien (6-Methylfulven), 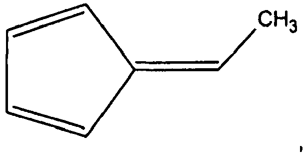 und
5-((1-Methylethyliden)-1,3-cyclopentadien (6,6-Dimethylfulven)

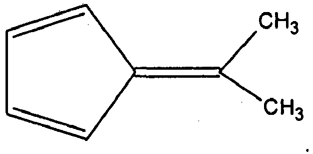

Im Algemeinen handelt es sich bei den Fulvenen um Stoffgruppen mit folgendem

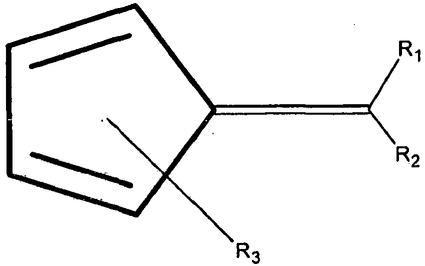

### Grundkörper:

mit R₁ = H, CH₃, C₂H₅, C₃H₇ oder C₄H₉
mit R₂ = H, CH₃, C₂H₅, C₃H₇ oder C₄H₉
mit R₃ = H, CH₃, C₂H₅, C₃H₇ oder C₄H₉

Diese Komponenten und andere, nicht-cyclische konjugierte Diene und Triene, sind hoch-ungesättigte Komponenten und absorbieren aufgrund ihres konjugierten Doppelbindungssystems Licht im sichtbaren Bereich. Daher sind diese Komponenten intensiv gefärbt. Sowohl die Fulvene als auch andere Cyclopentadien-Derivate als auch andere gefärbte konjugierten Systeme werden im folgenden als "Farbgeber" bezeichnet. Farbgeber zeichnen sich dadurch aus, dass ihre Moleküle chromophore Gruppen enthalten. Als "chromophore Gruppen" werden solche Atomgruppen innerhalb eines Moleküls bezeichnet, die Licht im sichtbaren Bereich absorbierten. Die Fulvene und andere Farbgeber bilden sich bei Dampfspaltung von Naphtha. Der Siedepunkt des Grundkörpers Fulven liegt bei Atmosphärendruck bei etwa 60 °C, der Siedepunkt des 6,6-Dimethylfulvens liegt bei etwa 160 °C.

Aufgrund der Siedepunkte der Farbgeber könnte man meinen, diese seien entweder bevorzugt in der Vordestillation mit der C₇₋-Fraktion (Schritt (1)) abzutrennen oder mit der Styrol-armen Raffinatfraktion in der Extraktivdestillation vom Styrol abzutrennen (Schritt (2)). Aber konjugierte Doppelbindungen, wie sie die oben erwähnten Farbgeber besitzen, sind in der Lage, über eine Diels-Alder-Reaktion mit Dienophilen, hier insbesondere Olefinen, zu reagieren. Die dabei gebildeten Verbindungen haben höhere Siedepunkte als die zur Reaktion gebrachten Komponenten. Die Produkte dieser Reaktion werden im folgenden "Diels-Alder-Produkte" genannt. Die Diels-Alder-Reaktion ist eine reversible Reaktion, die Diels-Alder-Produkte können daher wieder in die Ausgangsprodukte, hier die Verbindungen mit den konjugierten Doppelbindungen und das Olefin, zertallen.

Durch die Diels-Alder-Reaktion werden die Farbgeber "maskiert". Anfänglich leichtsiedende Farbgeber, die aufgrund ihres Siedepunktes in der Vordestillation mit der C₇₋-Fraktion abtrennbar hätten sein sollen, verbleiben in der C₈₊-Fraktion. Die C₈₊-Fraktion wird in der zweiten Destillationskolonne nochmals destilliert. Dabei spalten sich Teile der Diels-Alder-Produkte aufgrund der hohen Temperaturen und der relativ langen Verweilzeit der Sumpffraktion im Kolonnensumpf in die Farbgeber und die entsprechenden Dienophile zurück. Somit gelangen die Farbgeber in die C₈-Fraktion. Ein Teil der Farbgeber in der C₈-Fraktion reagiert wiederum mit Olefinen zu Diels-Alder-Produkten. Dies geschieht insbesondere bei langen Verweilzeiten, wie sie zum Beispiel in Rückflussbehältern oder Zwischenprodukt-Pufferbehältern herrschen. Diese weiteren Diels-Alder-Produkte maskieren die Farbgeber ein weiteres mal. So ist es möglich, dass die als Diels-Alder-Produkte maskierten Farbgeber in die Stripperkolonne der extraktiven Destillation gelangen und dort aufgrund der hohen Temperaturen, insbesondere im Abtriebsteil und im Sumpf der Kolonne, wieder in ihre Ausgangskomponenten, den Farbgebern und den Olefinen, gespalten werden. Ein Teil der im Roh-Pyrolysebenzin enthaltenen Farbgeber gelangt somit trotz ihrer Siedepunktslage in die Styrolfraktion aus der Extraktiv-Destillation und führt dort zu einer Färbung des Produkts.

Die Aufgabe der Erfindung ist es daher, ein Verfahren zur Gewinnung von farblosem Styrol mit Polymerisationsqualität aus Pyrolysebenzolfraktionen zur Verfügung zu stellen, welches ohne einen abschließenden Schritt zur Entfärbung des als Extrakt einer extraktiven Destillation anfallenden Reinstyrols auskommt und durch Einsparung von Aufbereitungsschritten eine wirtschaftlichere Herstellung ermöglicht.

Die Aufgabe löst diese Aufgabe entsprechend dem Hauptanspruch mittels extraktiver Destillation, indem
- die Pyrolysebenzolfraktion in einer Trennwandkolonne aufgetrennt wird in eine C₈-Kernfraktion, eine C₇₋-Fraktion und eine C₉₊-Fraktion,
- die erhaltene C₈-Kernfraktion einer selektiven Hydrierung des in ihr enthaltenen Phenylacetylens C₈H₆ unterworfen wird,
- anschließend eine extraktiv-destillative Auftrennung der erhaltenen C₈-Fraktion in eine Styrolfraktion und eine Styrol-arme Fraktion vorgenommen wird.

Durch den Einsatz der Trennwandkolonne ist es möglich, die leichtsiedenden Farbgeber in die C₇₋-Fraktion zu bringen, ohne dass die Möglichkeit der Maskierung als Diels-Alder-Produkt entsteht. Aufgrund der üblicherweise niedrigen Temperaturen in der Trennwandkolonne ist die Geschwindigkeit der exothermen Diels-Alder-Reaktion nur gering, so dass Diels-Alder-Produkte nur in sehr geringem Ausmaß gebildet werden. Diese geringen Mengen an gebildeten Diels-Alder-Produkten gelangen in den Kolonnensumpf und werden dort mit der C₉₊-Fraktion entfernt.

Trennwandkolonnen zum Einsatz in der extraktiven Destillation sind vorbekannt. Die WO2004052492 A1 beschreibt ein Verfahren zur Auftrennung eines Ausgangsgemisches aus zwei oder mehreren Komponenten durch Extraktivdestillation mit einem selektiven Lösungsmittel in einer Trennwandkolonne, wobei das Verfahren in einer Trennwandkolonne mit einer in Kolonnenlängsrichtung angeordneten Trennwand durchgeführt wird, die bis zum oberen Kolonnenende durchgezogen ist, so dass das Kolonneninnere in einen ersten Teilbereich, einen zweiten Teilbereich und einen unteren gemeinsamen Kolonnenbereich aufgeteilt wird, und das Ausgangsgemisch dem ersten Teilbereich zugeführt wird, und aus dem ersten Teilbereich ein erster Kopfstrom und aus dem zweiten Teilbereich ein zweiter Kopfstrom mit jeweils vorgegebener Spezifikation abgezogen wird, und das selektive Lösungsmittel im oberen Bereich des ersten und/oder des zweiten Teilbereichs aufgegeben wird, und die Menge des auf den ersten Teilbereich aufgebenen Lösungsmittelstromes dergestalt eingestellt wird, dass die jeweils vorgegeben Spezifikationen eingehalten werden.

Werden im Sumpf der Trennwandkolonne trotzdem Diels-Alder-Produkte in ihre Ausgangsprodukte zurückgespalten, so können sie aufgrund ihrer Siedepunkte nur in die C₇₋-Kopffraktion gelangen und nicht in den C₈-Seitenstrom. Damit ist auch jede Möglichkeit ausgeschlossen, dass sie das Endprodukt der extraktiven Destillation, das Reinstyrol, verfärben. Daher kann die Nachbehandlungsstufe (Schritt (5)) und die sich daran anschließende Destillation (Schritt (6)) aus dem herkömmlichen Stand der Technik entfallen, ebenso der Einsatz der hierin verwendeten Chemikalien.

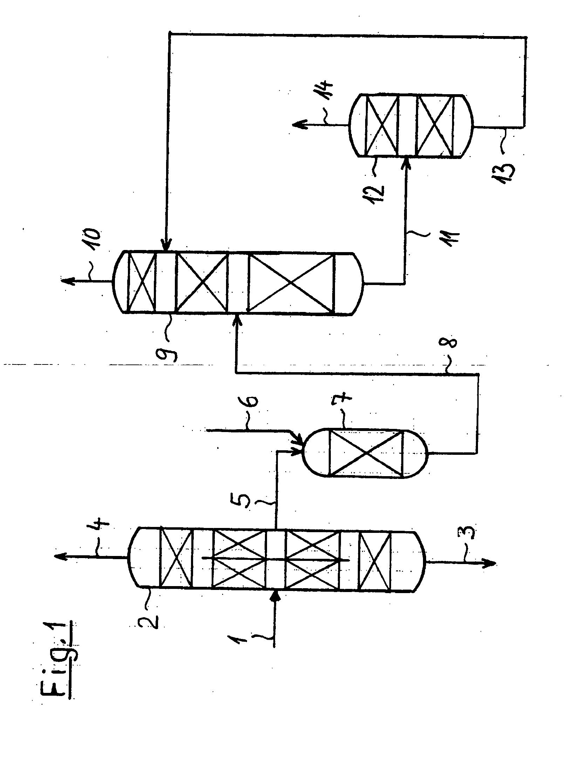

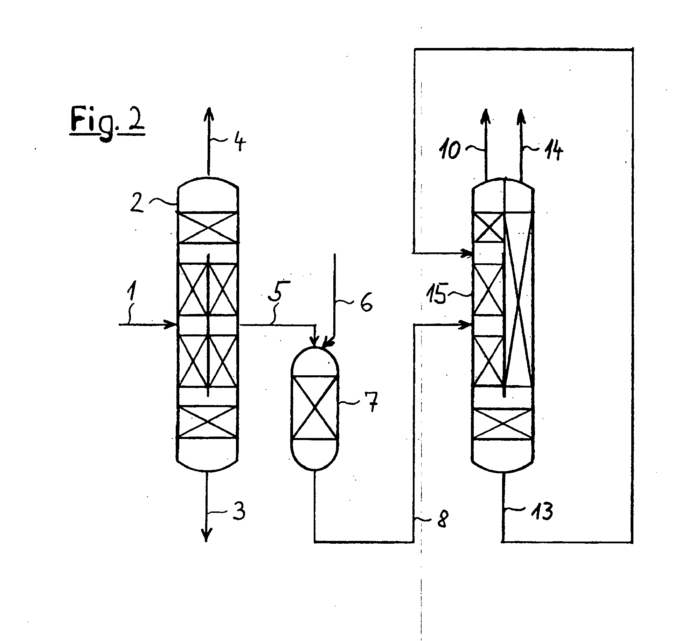

Die Erfindung wird im Folgenden an zwei Ausführungsbeispielen näher erläutert. Fig. 1 und 2 zeigen dabei vereinfachte Fließbilder des Verfahrens mit je einer Vordestillation in einer Trennwandkolonne, einer Selektivhydrierung und einer Extraktivdestillation, wobei die Extraktivdestillation in Fig. 1 auf konventionelle Weise und in Fig. 2 in einer weiteren Trennwandkolonne dargestellt ist.

Das Roh-Pyrolysebenzin 1 wird auf die den Einsatzboden enthaltene Seite der Trennwandkolonne 2 aufgeben, in der die Vordestillation durchgeführt wird. Als Sumpfprodukt wird die C₉₊-Fraktion 3, als Kopfprodukt die C₇₋-Fraktion 4, und als Mittelfraktion der C₈-Kernschnitt 5 abgezogen. Der C₈-Kernschnitt 5 wird zusammen mit Wasserstoff 6 einer Selektivhydrierung 7 unterzogen.

Fig. 1 zeigt, dass der aus der Selektivhydrierung 7 abgezogene hydrierte C₈-Kernschnitt 8 in eine Extraktivdestillationskolonne 9 geführt wird, von deren Kopf ein Styrol-abgereicherter C₈-Kernschnitt 10 abgezogen wird und ein im Extraktionsmittel gelöster Styrolstrom 11 aus dem Sumpf in die Stripperkolonne 12 geleitet wird, wo das Extraktionsmittel 13 zum Einsatz in der Extraktivdestillationskolonne 9 zurückgewonnen und das Reinstyrol 14 über Kopf abgeführt wird.

Fig. 2 zeigt alternativ, dass der aus der Selektivhydrierung 7 abgezogene hydrierte C₈-Kernschnitt 8 in eine Trennwand-Extraktivdestillationskolonne 15 mit oberseitig geschlossener rechter Seite im Trennwandbereich geführt wird, wobei der Abtreiber in den Sumpf sowie die rechte Seite der Trennwandkolonne integriert ist, das Reinstyrol 14 aus dem Seitenabzug des rechten Trennwandbereichs abgezogen, das zurückgewonnene Extraktionsmittel 13 oberhalb des den Kohlenwasserstofffeedboden enthaltenen Trennwandteil aufgegeben und im Kopf der Trennwand-Extraktivdestillationskolonne 15 ein Styrol-abgereicherter C₈-Kernschnitt 10 abgezogen werden.

Für die einzelnen Verfahrensschritte im erfindungsgemäßen Verfahren werden in Tabelle 1 die typischen Prozessbedingungen angegeben:

| **Tabelle 1** | Parameterbereich | Bevorzugter Parameterbereich | Beispiel |
|---|---|---|---|
| **Vordestillation Trennwandkolonne** | | | |
| Temperatur am Kopf | 50 - 120 °C | 50 - 100°C | 100°C |
| Temperatur im Sumpf | 100 - 150 °C | 120 - 140 °C | 128 °C |
| Druck am Kopf | 100 - 400 mbar | 200 - 300 mbar | 300 mbar |

| **Selektivhydrierung** | | | |
|---|---|---|---|
| Reaktionstemperatur | 20 - 60 °C | 20 - 40 °C | 25 °C |
| Partialdruck Wasserstoff | 1 - 20 bar | 5 - 10 bar | 8 bar |
| Raumgeschwindigkeiten LHSV | 1 - 20 L/(L·h) | 5 - 10 U(L.h) | 8 U(L·h) |
| Betriebsweise | | Flüssigphase | Flüssigphase |
| Katalysator | | Edelmetallbasiert | Pd auf Al₂O₃ |

| **Extraktiv-Destillation (gemäß** **Fig. 1****)** | | | |
|---|---|---|---|
| Temperatur am Kopf | 20 - 120 °C | 50 - 80 °C | 77 °C |
| Temperatur im Sumpf | 100 - 150 °C | 120 - 140 °C | 129 °C |
| Druck am Kopf | 10 - 400 mbar | 50 - 200 mbar | 90 mbar |
| Umlaufverhältnis | 1 - 20 kg Lösemittel / kg Feed | 5 - 15 kg Lösemittel / kg Feed | 8,8 kg Lösemittel / kg Feed |

| **Stripper-Kolonne (gemäß** **Fig. 1****)** | | | |
|---|---|---|---|
| Temperatur am Kopf | 20 - 120 °C | 50 - 80 °C | 67 °C |
| Temperatur im Sumpf | 100 - 180 °C | 140 - 160 °C | 150 °C |
| Druck am Kopf | 10 - 400 mbar | 50 - 200 mbar | 80 mbar |

| **Extraktiv-Destillation (gemäß** **Fig. 2****)** | | | |
|---|---|---|---|
| Temperatur am Kopf (Raffinatseite) | 20 - 120 °C | 50 - 80 °C | 67 °C |
| Temperatur am Kopf Extraktseite) | 20 - 120 °C | 50 - 80 °C | 70 °C |
| Temperatur im Sumpf | 100 - 180 °C | 120 - 160 °C | 150°C |
| Druck am Kopf | 10 - 400 mbar | 50 - 200 mbar | 80 mbar |

| | | | |
|---|---|---|---|
| Alle Druckangaben als Absolutdruck! | | | |

## Patentansprüche

1. Verfahren zur Gewinnung von Styrol mit Polymerisationsqualität aus Styrol enthaltenden Pyrolysebenzolfraktionen mittels extraktiver Destillation, **dadurch gekennzeichnet, dass**
• die Pyrolysebenzolfraktion in einer Trennwandkolonne aufgetrennt wird in eine C₈-Kernfraktion, eine C₇₋-Fraktion und eine C₉₊-Fraktion,
• die erhaltene C₈-Kernfraktion einer selektiven Hydrierung des in ihr enthaltenen Phenylacetylens C₈H₆ unterworfen wird,
• anschließend eine extraktiv-destillative Auftrennung der erhaltenen C₈-Fraktion in eine Styrolfraktion und eine Styrol-arme Fraktion vorgenommen wird.

## Claims

1. Process for the production of styrene of polymerisation grade from pyrolysis benzene fractions containing styrene by extractive distillation,
**characterised in that**
• the pyrolysis benzene fraction is fractionated in a divided-wall column so as to obtain a C₈ core fraction, a C₇₋ fraction and a C₉₊fraction;
• the C₈ core fraction thus obtained undergoes a selective hydrogenation of the phenylacetylene C₈H₆ contained in the said core fraction;
• an extractive distillation of the C₈ core fraction obtained is carried out to extract a styrene fraction and a fraction poor in styrene.

## Revendications

1. Procédé pour obtenir du styrolène présentant des qualités de polymérisation depuis des fractions de benzène de pyrolyse contenant du styrolène au moyen d'une distillation extractive, **caractérisé en ce que**
■ la fraction de benzène de pyrolyse est séparée dans une colonne à cloison en une fraction essentielle C₈, en une fraction C₇₋ et une fraction C₉₊,
■ la fraction essentielle C₈ obtenue est soumise à une hydrogénation sélective du phénylacétylène C₈H₆ qu'elle contient,
■ et a lieu ensuite par distillation extractive une séparation de la fraction C₈ en fraction de styrolène et une fraction pauvre en styrolène.
